# FASCICULE DE BREVET EUROPEEN

(11) **EP 1 948 085 B1**
(45) Date de publication et mention de la délivrance du brevet: **03.06.2015**
(21) Numéro de dépôt: 06831098.6
(22) Date de dépôt: 09.11.2006
(51) Int. Cl.: A61F 2/16

(54) **INJECTEUR D'IMPLANT OPHTALMIQUE**
INJEKTIONSVORRICHTUNG FÜR OPHTHALMISCHES IMPLANTAT
OPHTHALMIC IMPLANT INJECTOR

(30) Priorité: 09.11.2005 FR 0511421; 16.03.2006 FR 0602323
(43) Date de publication de la demande: 30.07.2008
(73) Titulaire: SEDAT, 69540 Irigny (Rhône) (FR)
(72) Inventeur: PESSIN, Olivier, F-69290 Grezieu La Varenne (FR)
(74) Mandataire: Blot, Philippe Robert Emile
(86) Numéro de dépôt international: PCT/FR2006/002498
(87) Numéro de publication internationale: WO 2007/054644

(56) Documents cités:
- EP-A- 1 502 559
- WO-A-96/28121
- WO-A-97/15253
- WO-A-03/045285
- DE-U1- 20 219 445
- DE-U1-202004 017 931
- US-A- 5 496 328
- US-A1- 2005 154 399

## Description

La présente invention concerne un injecteur d'implant ophtalmique comportant :
- une cartouche de pliage contenant un implant à injecter, la cartouche comprenant une canule d'injection de l'implant et une excroissance radiale de liaison axiale ; et
- un mécanisme d'injection comprenant un corps tubulaire présentant à une extrémité des moyens d'accrochage de la cartouche, lesquels moyens d'accrochage de la cartouche comportent une encoche ménagée dans le corps tubulaire et présentant à son extrémité débouchant à l'extrémité du corps un passage étroit prolongé par une lumière évasée permettant une liaison par baïonnette de la cartouche et du corps tubulaire.

Il est connu pour le traitement de la cataracte qu'un chirurgien injecte dans l'oeil du patient un implant ophtalmique ayant par exemple la forme d'une lentille équipée latéralement de deux bras arqués diamétralement opposés (par example WO-9628121).

L'implant est injecté dans l'oeil du patient par l'intermédiaire d'une canule d'injection, en étant poussé par un piston au travers de cette canule. Pour le passage au travers de la canule, l'implant est enroulé sur lui-même en spirale autour d'une direction diamétrale reliant les deux bras de l'implant.

L'enroulement de l'implant sous forme de spirale est désigné par pliage et est effectué dans une cartouche de pliage. Cette cartouche comporte la canule d'injection et est adaptée pour être rapportée après chargement sur un mécanisme d'injection comportant un corps de support et un poussoir coulissant.

L'accouplement de la cartouche à l'extrémité du corps réalisé par exemple par un agencement à baïonnette. Ainsi, une encoche est ménagée à l'extrémité avant du corps et la cartouche comporte une excroissance radiale permettant sa liaison par engagement dans l'encoche.

Afin d'assurer le verrouillage de l'engagement de la baïonnette, il est connu de prévoir une bague fendue montée mobile à rotation autour du corps. Cette bague est portée par le corps et est propre à recevoir l'excroissance radiale de la canule et à déplacer celle-ci en rotation pour l'amener au fond de l'encoche en regard d'un épaulement assurant un biocage axial de la canule par rapport au corps.

Un tel mécanisme est relativement complexe à fabriquer et augmente le coût de l'injecteur.

L'invention a pour but de proposer un injecteur d'implant d'un coût de fabrication réduit.

A cet effet, l'invention a pour objet un injecteur du type précité, caractérisé en ce que l'injecteur comporte un étui de protection de la canule de la cartouche de pliage, lequel étui comporte un boîtier de réception de l'extrémité d'injection de la canule, et au moins une surface s'étendant radialement d'appui de l'excroissance radiale contre l'étui.

Selon des modes particuliers de réalisation, l'injecteur comporte l'une ou plusieurs des caractéristiques suivantes :
- la cartouche de pliage comporte une pince formée de deux branches articulées présentant chacune une aile de manoeuvre propres à être accolées et làdite excroissance radiale est formée des deux ailes de manoeuvre accolées ;
- l'étui de protection comporte deux saillies et la canule présente deux encoches de positionnement propres à coopérer avec les saillies pour le positionnement relatif de l'étui et de la canule ;
- la longueur du boîtier de l'étui est supérieure à la longueur de la canule ;
- le boîtier de réception de l'extrémité d'injection de la canule comporte deux surfaces d'appui de l'excroissance radiale contre l'étui ;
- le boîtier présente une ouverture d'introduction de la canule qui est bordée sur plus de la moitié de son pourtour par une lèvre présentant à chaque extrémité une surface d'appui ;
- la ou chaque surface d'appui s'étend autour du corps lorsque la cartouche contenue dans l'étui est engagée à l'avant du corps ;
- l'étui est déplaçable angulairement par rapport au corps lorsque la cartouche contenue dans l'étui est engagée à l'avant du corps ;
- le corps est dépourvu de bague rotative pour la mise en place de l'excroissance radiale dans l'encoche ; et
- le corps comporte une languette élastique de sollicitation de l'excroissance radiale de la cartouche en regard d'un épaulement bordant la lumière évasée de l'encoche.

Selon des modes particuliers de réalisation, la cartouche comporte l'une ou plusieurs des caractéristiques suivantes :
- elle comprend une pince formée de deux branches articulées présentant des canaux sensiblement coaxiaux bordés chacun par deux rives longitudinales, les canaux s'ouvrant en regard l'un de l'autre et délimitant lorsque les deux branches sont accolées un conduit de confinement de l'implant partiellement replié et en position accolée des deux branches, au moins deux des rives en regard des deux canaux sont décalés transversalement au conduit dans le plan de jonction des deux branches de sorte qu'une branche délimite un épaulement d'appui de l'implant faisant saillie en regard du canal ménagé dans l'autre branche ;
- l'épaulement a dans le plan de jonction une largeur moyenne mesurée transversalement à la direction des canaux comprise entre 0,1 et 0,8 mm ;
- les deux rives décalées sont plus éloignées de l'axe d'articulation des deux branches que les deux autres rives, lesquelles autres rives sont confondues lorsque les deux branches sont accolées ;
- la cartouche comporte une canule d'injection à surface interne convergente dans le prolongement du conduit de confinement de l'implant lorsque les deux branches sont accolées ;
- les deux canaux sont de section sensiblement semi-circulaire ou semi-elliptique ;
- l'épaulement est sensiblement plan et l'axe du conduit formé des deux canaux s'étend sensiblement dans le plan défini par l'épaulement ;
- chaque branche présente au-delà du canal, du côté opposé à l'axe d'articulation, une aile de manoeuvre et l'épaulement prolonge une aile de manoeuvre de l'une des branches ; et
- les deux branches comportent, au voisinage des rives des canaux, en dehors des canaux, des profils d'emboîtement complémentaires en saillie et en creux dimensionnés de sorte que les profils sont emboîtés lorsque les deux branches sont accolées.

L'invention a également pour objet un injecteur d'implant comportant une cartouche telle que définie ci-dessus et un mécanisme d'injection comprenant un corps de liaison à la cartouche et un poussoir propre à pousser l'implant contenu dans la cartouche.

L'invention sera mieux comprise à la lecture de la description qui va suivre, donnée uniquement à titre d'exemple et faite en se référant aux dessins sur lesquels :
- les Figures 1 et 1A sont des vues en perspective de trois-quarts respectivement arrière et avant d'un injecteur d'implant ophtalmique équipé d'une cartouche prêt pour une injection ;
- la Figure 1B est une vue en perspective du corps de l'injecteur en l'absence de cartouche ;
- la Figure 2 est une vue en perspective de l'injecteur de la Figure 1, la cartouche étant partiellement recouverte de son étui de protection ;
- la Figure 3 est une vue en perspective éclatée de la cartouche de pliage ouverte, de l'étui de protection et du piston ;
- la Figure 4 est une vue en perspective de la cartouche de pliage reçue partiellement dans l'étui avec les deux branches ouvertes et un implant avant-déformation maintenu entre les deux branches;
- la Figure 5 est une vue en coupe transversale de la cartouche prise suivant le plan V-V de la Figure 4 ;
- la Figure 6 est une vue en coupe de la cartouche prise suivant la ligne VI-VI de la Figure 7 ;
- la Figure 6A est une vue agrandie d'une partie de la Figure 6 ;
- la Figure 7 est une vue en élévation de la cartouche de pliage refermée ;
- la Figure 8 est une vue en section de la cartouche prise suivant la ligne VIII-VIII de la Figure 6 lors du déplacement de l'implant ;
- la Figure 9 est une vue en section transversale de la cartouche de pliage, lors du rapprochement des deux branches montrant un mauvais positionnement de l'implant ; et
- la Figure 10 est une vue identique à celle de la Figure 1B d'une variante de réalisation du corps de l'injecteur.

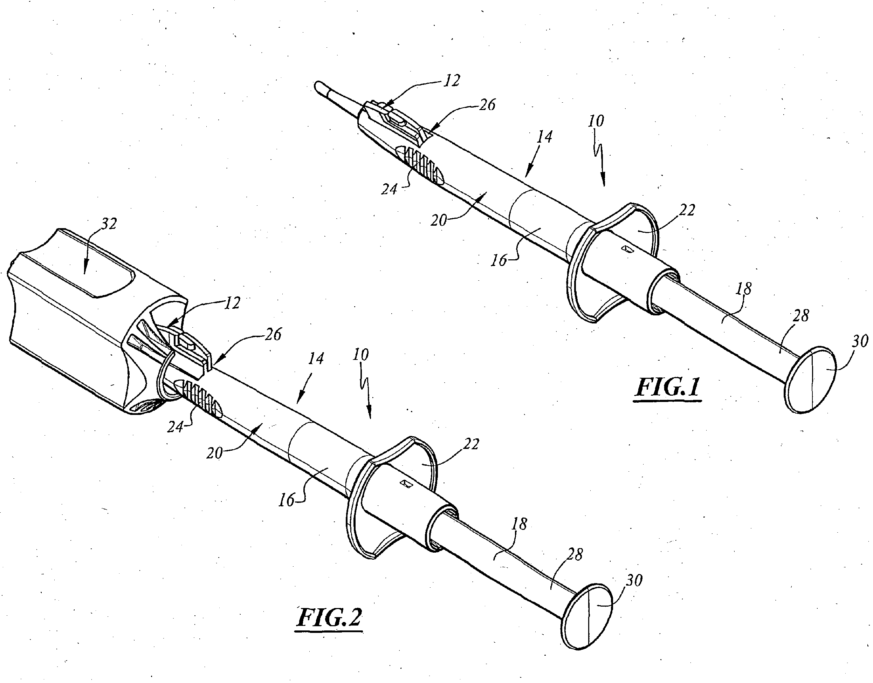

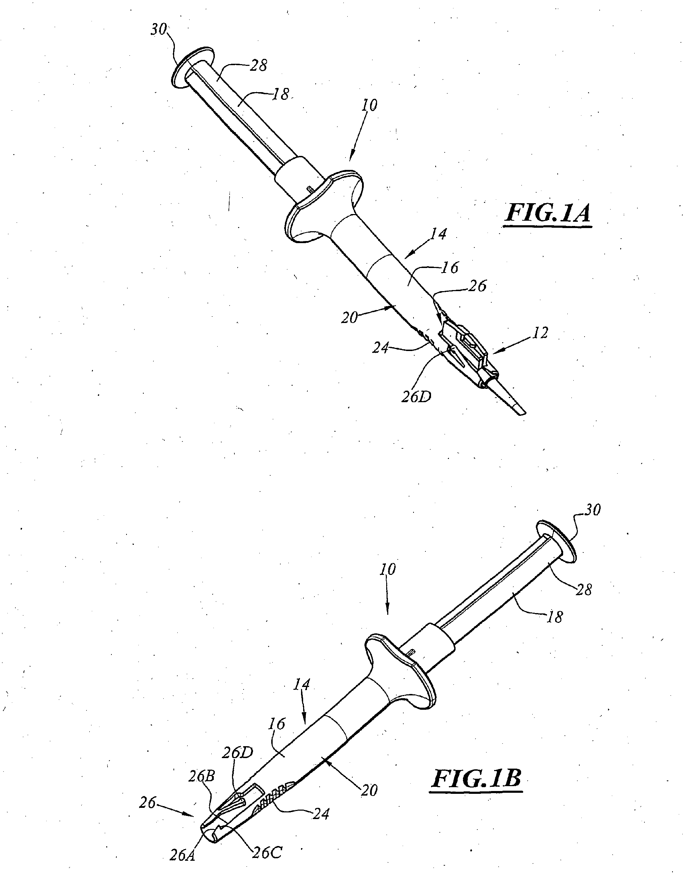

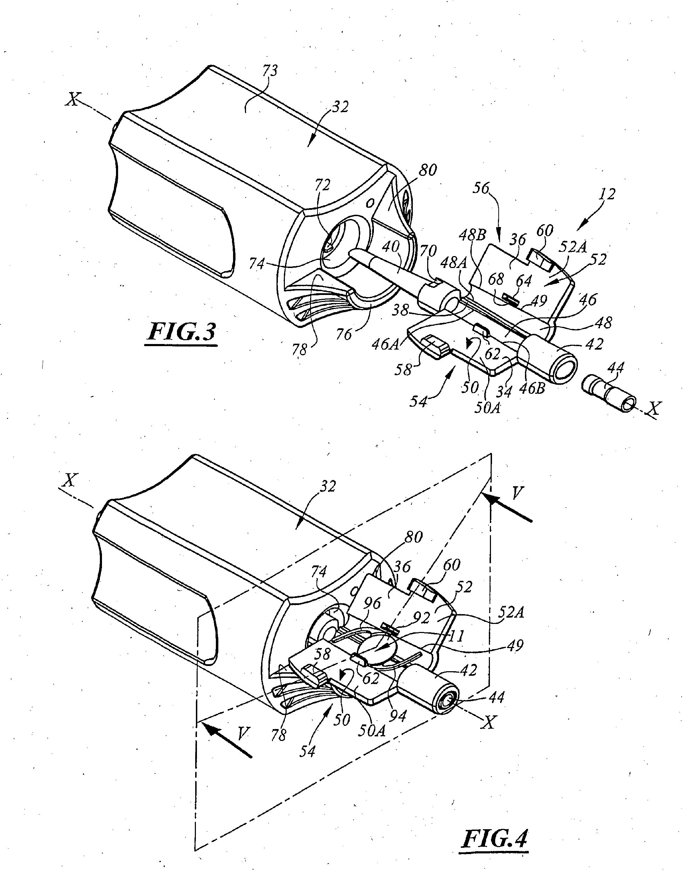

L'injecteur d'implant 10 représenté sur la Figure 1 est destiné à introduire dans l'oeil du patient un implant ophtalmique 11 visible sur la Figure 4 et comprenant notamment une lentille ayant généralement la forme d'un disque.

Comme connu en soi, l'injecteur comporte une cartouche de pliage 12 contenant l'implant à injecter et un mécanisme d'injection 14 formé d'un corps tubulaire 16 présentant à une extrémité des moyens d'accrochage de la cartouche de pliage et un poussoir 18 monté coulissant au travers du corps dans le prolongement de la cartouche de pliage.

Plus précisément, le corps 16 comporte un tube 20 présentant à une extrémité arrière une collerette périphérique 22 formant un appuie-doigt. A son extrémité opposée, le tube 20 présente des empreintes anti-dérapantes 24 facilitant sa préhension. Le corps tubulaire 16 est fendu à son extrémité opposée à celle par laquelle pénètre le poussoir 18 d'une encoche 26 visible sur la Figure 1A propre à recevoir une excroissance radiale de la cartouche de pliage 12 et permettant une liaison axiale de la cartouche 12 et du corps 16. Ainsi, l'encoche 26 présente à son extrémité débouchant à l'extrémité du tube 20 un passage étroit 26A prolongé par une lumière évasée 26B permettant une liaison par baïonnette de la cartouche 12 et du corps 16.

Le passage étroit 26A est séparé de la lumière évasée par un épaulement 26C orienté à l'opposé de l'extrémité débouchante de l'encoche 26. Une languette élastique 26D, venue de matière avec le corps fait saillie dans la lumière évasée. Elle est propre à repousser l'excroissance radiale de liaison de la cartouche du côté de la lumière délimitant l'épaulement 26C. Cette languette est orientée vers l'arrière du corps, de sorte que celle-ci est déformable élastiquement lors de l'insertion de l'excroissance de liaison dans l'encoche 26.

Le poussoir 18 comporte une tige d'actionnement 28 propre à traverser de part en part le tube 20 et à l'extrémité arrière de la tige 28 une pastille 30 formant appuie-doigt.

Comme illustré sur la Figure 2, l'injecteur comporte en outre un étui de protection 32 de l'extrémité d'injection de la cartouche de pliage. Cet étui est visible avec la cartouche ouverte sur la Figure 3.

La cartouche 12 comporte deux branches 34, 36 articulées l'une par rapport à l'autre autour d'une charnière 38 dont l'axe s'étend parallèlement à l'axe X-X d'injection de l'implant défini par la cartouche. Les deux branches articulées sont prolongées suivant l'axe d'injection X-X d'un côté par une canule 40 et de l'autre côté par un manchon 42 de réception d'un piston d'injection 44 propre à être déplacé suivant l'axe X-X sous l'action du poussoir 18.

Plus précisément, les deux branches 34, 36 présentent chacune au voisinage de l'articulation 38 un canal 46, 48. Ces deux canaux s'étendent parallèlement l'un à l'autre et s'ouvrent l'un en regard de l'autre lorsque les deux branches sont accolées. Ces canaux s'étendent parallèlement à la direction d'articulation de la charnière. Ils ont chacun un section semi-circulaire ou plus précisément semi-elliptique.

Les deux branches sont articulées de sorte que lorsque les branches sont écartées, les deux canaux 46, 48 s'étendent sensiblement parallèlement l'un à l'autre et délimitent transversalement deux cavités successives. En revanche, lorsque les deux branches 34, 36 sont accolées, les deux canaux 46, 48 se superposent pour former un conduit délimitant un contour fermé.

Les canaux 46, 48 sont délimités longitudinalement par des rives sensiblement parallèles. Ainsi, chaque canal est délimité par une rive intérieure 46A, 48A ménagée le long de la charnière 38. Ces rives s'étendent parallèlement l'une à l'autre et sont telles que lorsque les deux branches 34, 36 sont accolées, les deux rives intérieures 46A, 48A sont rigoureusement alignées et confondues, les deux canaux se prolongeant continûment et , tangentiellement au niveau des rives intérieures accolées 46A, 48A.

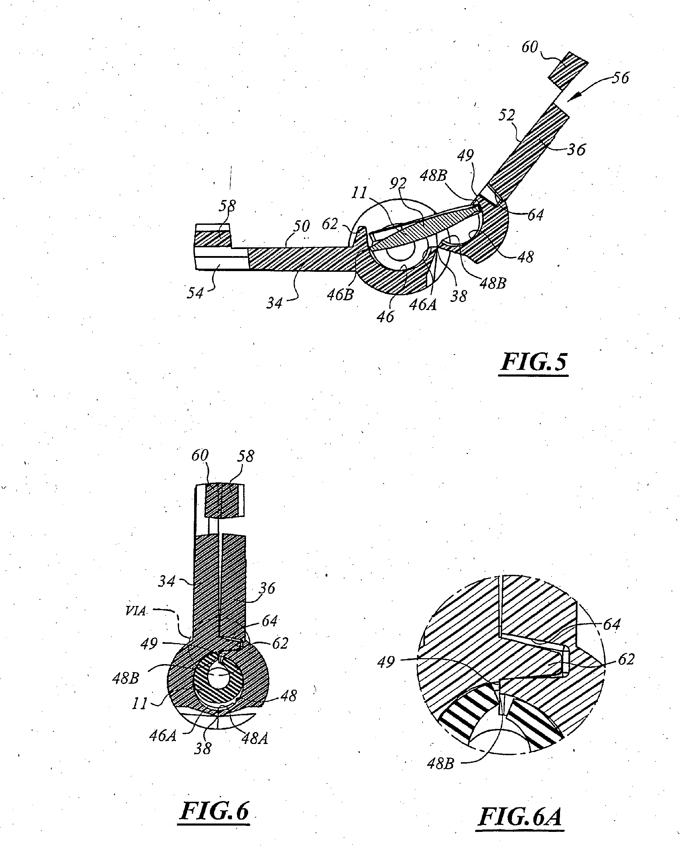

Les deux canaux 46, 48 sont de l'autre côté délimités par deux rives extérieures 46B, 48B ménagées à distance de l'articulation 38. Les rives extérieures 46B, 48B sont décalées transversalement par rapport à l'axe du conduit comme cela sera décrit en détail dans la suite de sorte que lorsque les deux branches sont accolées, les deux rives extérieures 46B, 48B sont décalées l'une à l'autre sans être confondues en formant entre elles un épaulement 49 visible sur la Figure 6A.

En particulier, les deux rives 46B, 48B sont concourantes lorsque les deux branches sont accolées en un point d'extrémité situé immédiatement en sortie du manchon 42. La rive 48B diverge de la rive 46B en direction dé la canule 40 en s'écartant de la rive 48B vers l'intérieur du conduit défini par les deux canaux.

Ainsi, l'épaulement 49 est généralement triangulaire ou tropézoïdale, sa largeur augmentant en direction de la canule 40.

Avantageusement, l'épaulement 49 a une largeur moyenne mesurée perpendiculairement à la direction des canaux 46, 48 comprise entre 0,1 mm et 0,8 mm. Plus précisément, et compte tenu de sa forme triangulaire, sa largeur varie de 0 mm au voisinage du manchon 42 à une valeur comprise entre 0,2 et 1 mm et notamment égale à 0,4 mm au niveau de la canule 40.

L'épaulement 49 est plat. Pour ménager l'épaulement 49, les deux canaux, ont une largeur différente, les rives 46A, 46B du canal 46 ayant un écartement supérieur aux rives 48A, 48B du canal 48 à l'écart du manchon 42.

Chaque branche 34, 36 présente au-delà du canal 46, 48 associé du côté opposé à la charnière 38 une aile de manoeuvre 50, 52. Ces deux ailes sont de forme générale rectangulaire. Elles présentent l'une en regard de l'autre des surfaces d'appui planes 50A, 52A propres à s'appliquer l'une sur l'autre lorsque les deux branches sont accolées. Ces ailes s'étendent radialement par rapport à la charnière 38. L'épaulement 49 s'étend dans le prolongement de la surface plane 52.

Les deux ailes de manoeuvre, une fois accolées forment l'excroissance de liaison propre à être reçue dans l'encoche 26 comme illustré sur les Figures 1 et 1A.

Suivant leur bord libre opposé à la charnière 38, les ailes 50, 52 comportent des encoches respectives 54, 56 disposées tête-bêche. A l'inverse, les ailes 50, 52 comportent sur la surface d'appui 50A, 52A, au droit de l'encoche de l'aile complémentaire des saillies d'encliquetage 58, 60. Ces saillies sont propres à être reçues dans les encoches 54, 56 ménagées sur l'aile en regard. Elles présentent suivant leur extrémité en regard des profils d'enclenchement élastique complémentaires présentant des profils associés en saillie et en creux permettant un maintien des deux branches accolées l'une sur l'autre, les canaux constituant ensemble un conduit à section fermée.

Le long des rives extérieures 46B, 48B des canaux, dans la partie médiane des ailes 50, 52 est prévue une saillie 62 ménagée sur l'aile 50 et une cavité de forme correspondante 64 ménagée dans l'aile 52 est propre à recevoir la saillie 62.

La saillie 62 prolonge la surface du canal 46 à laquelle elle est reliée tangentiellement.

En revanche, la cavité 64 est ménagée à distance de la rive 48B de sorte qu'une partie 66 de l'épaulement 49 s'étend entre la cavité 64 et le canal 48.

Le piston 44 est fixé à l'extrémité du poussoir 18 et est propre à circuler au travers du manchon 42, du conduit délimité par les deux canaux 46, 48 et de la canule 40.

Le manchon 42 et la canule 40 sont ménagés de part et d'autre du canal 42. Elles prolongent celui-ci à chaque extrémité et s'étendent suivant l'axe X-X du canal.

Le conduit intérieur du manchon 42 est de section sensiblement circulaire et constante sur sa longueur.

Le conduit intérieur délimité par la canule 40 est convergeant des canaux 46, 48 vers l'extrémité libre de la canule. Ainsi, intérieurement, la canule présente une surface tronconique de section décroissante des canaux 46, 48 vers l'extrémité d'injection.

Extérieurement, la canule 40 présente deux encoches 70 de positionnement propres à coopérer avec des saillies 72 ménagées dans l'étui de protection.

L'étui de protection 32 est constitué d'un boîtier 73 présentant une ouverture 74 d'introduction de la canule 40 ménagée au centre d'une face d'extrémité. La longueur du boîtier est supérieure à la longueur de la canule. Extérieurement, l'ouverture 74 est bordée sur plus de la moitié de son pourtour par une lèvre 76 présentant à chaque extrémité des surfaces d'appui 78, 80 s'étendant radialement par rapport à l'ouverture 74 et propre à recevoir les ailes 50, 52 de la cartouche lorsque les branches 34, 36 sont écartées comme cela est illustré sur la Figure 4.

La cartouche de pliage 12 est illustrée en position ouverte sur les Figures 4 et 5 alors qu'un implant 11 est disposé entre les canaux 46, 48. Un tel implant présente comme connu en soi une lentille 92 constituée d'un disque transparent et deux bras arqués 94, 96 symétriques l'un de l'autre par rapport à l'axe de la lentille. Les deux bras sont diamétralement opposés par rapport à la lentille.

L'implant est formé dans un matériau polymère déformable élastiquement.

L'implant est mis en forme alors que la canule de la cartouche est maintenue dans l'étui de protection, les deux ailes s'appuyant sur les surfaces 78, 80. La lentille est placée en appui dans les canaux 46, 48 dans leur partie médiane, c'est-à-dire dans la région de la saillie 62 et de l'évidement 64, les deux bras 94, 96 étant disposés de part et d'autre respectivement du côté du manchon 42 et de la canule 40.

L'implant est représenté dans cette position sur la Figure 4.

Dans cette position, l'implant 11 est en contact de part et d'autre avec les surfaces des canaux 46, 48 et prend en appui sur ceux-ci en des points sensiblement diamétralement opposés.

On conçoit que le rapprochement des deux branches 34, 36 par basculement autour de l'articulation 38 provoque le repliement de l'implant 11 autour d'un axe parallèle à l'axe des canaux 46, 48 lorsque ce repliement est initié par le chirurgien appuyant par exemple au moyen d'une pince au centre de l'implant. En particulier, la partie centrale de la lentille se trouve appliquée sur la charnière 38 alors que les bords opposés de la lentille se trouvent rapprochés l'un de l'autre au contact des canaux 46, 48 comme illustré sur la Figure 6 lorsque les deux branches sont accolées.

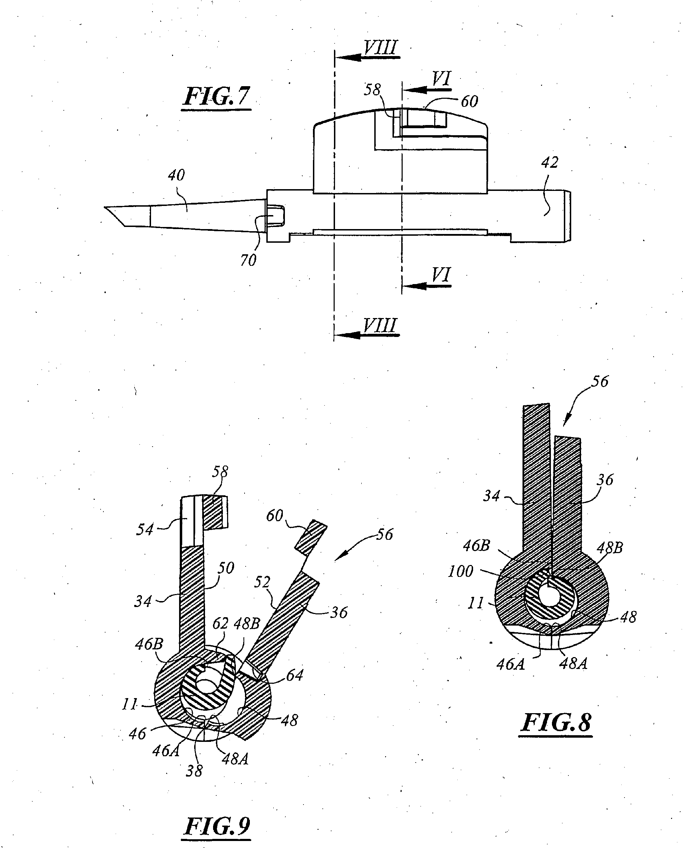

Dans la position et comme illustré sur la Figure 7, les deux branches sont maintenues accolées par coopération des profils d'accrochage 58, 60.

Dans la position de la Figure 6, le bord de la lentille du côté du canal 46 prend appui sur la plage de l'aile 52 formant l'épaulement 49 et faisant saillie à l'intérieur du conduit délimité par les deux canaux 46, 48. En revanche, l'extrémité de la lentille en appui contre le canal 48 se trouve fléchi légèrement à l'intérieur de l'extrémité opposée de la lentille, préfigurant ainsi l'enroulement de la spirale de la lentille.

Comme illustré sur la Figure 8, l'épaulement 49 s'étend sur l'essentiel de la longueur des canaux 46, 48. Ainsi, lorsque la lentille 92 est avancée sous l'action du piston 44 au travers du conduit, les bords opposés de la lentille se chevauchent en garantissant que le bord en appui sur l'épaulement 49 se trouve à l'extérieur du bord recourbé par le canal 48 grâce à l'épaulement 49.

La présence de l'épaulement 49 assure que lors de l'enfoncement de l'implant au travers de la canule, et lors de la poursuite de l'enroulement en spirale de l'implant, les bords opposés soient correctement décalés radialement et superposés, permettant un enroulement satisfaisant de l'implant sur lui-même, sans que les deux bords ne s'appuient l'un sur l'autre. De plus, le sens de roulement de l'implant est garanti puisque imposé par la forme des canaux 46, 48 et la présence de l'épaulement 49.

On conçoit par ailleurs que la présence de la saillie 62 et de l'évidement complémentaire 64 assure qu'en cas de mauvais positionnement initial de l'implant, il soit difficile d'accoler les deux ailes l'une à l'autre comme illustré sur la Figure 9. En effet, si l'un des bords de l'implant s'échappe de l'un des canaux 46, 48, ce bord constitue une barrière interposée entre la saillie 62 et la cavité 64 interdisant que la saillie pénètre dans l'évidement. Ainsi, le chirurgien sent un point dur lors de sa tentative de rapprochement des deux branches et est ainsi informé du mauvais positionnement de l'implant.

En revanche, en cas de positionnement correct de l'implant, comme illustré sur la Figure 7, la saillie 62 est reçue complètement dans la cavité 64.

Une fois l'implant replié et correctement positionné dans la cartouche, la cartouche est mise en place à l'extrémité du corps de l'injecteur. Pour ce faire, alors que la cartouche est supportée par l'étui de protection dans laquelle elle est maintenue, la canule étant engagée dans le corps de l'étui, la cartouche est amenée à l'extrémité avant du corps de l'injecteur et les deux ailes sont introduites dans l'encoche 26. Ce faisant, elle repousse la languette 26D, jusqu'à se trouver dans la partie évasée de l'encoche. L'étui et la cartouche solidarisés en rotation par, d'une part, les encoches 70 et les saillies 72 et, d'autre part, l'appui de l'excroissance radiale 50, 52 sur une surface d'appui 78, 80 sont alors tournés angulairement par rapport au corps de l'injecteur autour de leur axe pour amener l'excroissance radiale 50, 52 en regard de l'épaulement 26C.

Dans cette position, la languette 26D repousse l'excroissance radiale contre le fond de l'encoche et maintient celle-ci en regard de l'épaulement 26C évitant tout déplacement accidentel de l'étui par rapport au corps.

Lors du déplacement de l'étui 32 autour du corps, l'extrémité avant du corps est engagée dans l'étui, et notamment recouverte par la lèvre 76 reliant les deux surfaces d'appui 78, 80. Une fois la cartouche en position, l'étui est retiré axialement par traction. Il laisse alors en place la cartouche à l'extrémité du corps, la canule 40 étant exposée et faisant saillie axialement à l'extrémité du corps.

On comprend qu'un tel injecteur est d'un coût de fabrication réduit puisque aucune pièce mobile, telle qu'une bague n'est nécessaire ni prévue à l'extrémité avant du corps pour permettre la mise en place de la cartourche et le maintien de celle-ci. En effet, la cartouche est mise en place grâce à l'action de l'étui, lequel assure à la fois la protection de la canule et le guidage de la cartouche lors de son accouplement par baïonnette. La languette 26D assure la retenue en position de la cartouche.

Pendant toute la phase de mise en place de cartouche sur le corps de l'injecteur, la cartouche, est notamment la canule de la cartouche est protégée dans l'étui et n'est donc pas exposée.

Dans ce mode de réalisation, la cartouche peut être retirée du corps de l'injecteur après mise en place de la lentille. Pour ce faire, l'étui est à nouveau utilisé. Celui-ci est engagé sur l'extrémité avant de la cartouche jusqu'à ce que l'étui et la cartouche soient à nouveau solidarisés en rotation. L'étui est alors décalé angulairement par rapport à l'axe de la cartouche vers la languette élastique 26D, entraînant avec lui la cartouche. L'excroissance de la cartouche est alors amenée en regard du passage étroit 26A, la languette 26D étant comprimée. La cartouche est ensuite tirée axialement pour être extraite de l'encoche 26.

Ainsi, dans ce mode de réalisation, la cartouche est montable et démontable à l'aide de l'étui sans que celle-ci ne soit à aucun moment exposée, évitant ainsi les risques d'endommagement.

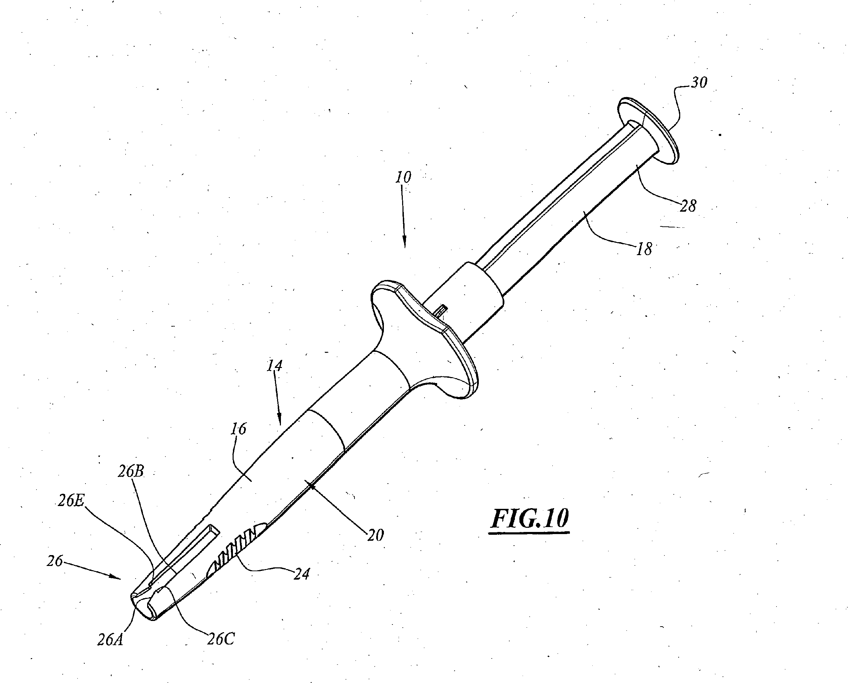

Sur la Figure 10 est représentée une autre variante de réalisation du corps de l'injecteur. Dans cette variante, la languette 26D est supprimée et l'encoche 26 s'évase au-delà du passage étroit 26A de part et d'autre de l'axe de ce passage pour délimiter deux épaulements symétriques 26C et 26E.

De préférence, le passage étroit 26A est de section progressivement décroissant vers la lumière évasée 26B et la section minimale du passage étroit est très légèrement inférieure à l'épaisseur correspondante de l'excroissance radiale formée des ailes de manoeuvre accouplées de la cartouche.

Avantageusement, les deux épaulements 26C, 26E sont inclinés vers l'intérieur de la lumière 26B du côté de leur extrémité libre. Ainsi, ces deux épaulements sont légèrement rentrants ou en contre-dépouille formant des harpons de retenue de l'excroissance radiale de la cartouche.

Dans ce mode de réalisation, la cartouche mise en place grâce à l'étui peut être, après engagement dans la lumière 26B, déplacée angulairement d'un côté ou dans l'autre. Elle est dans les deux cas retenue par les épaulements 26C et 26E formant harpon.

Compte tenu de la présence des deux épaulements et de la largeur réduite du passage étroit 26A, la cartouche ne peut pas dans ce mode de réalisation être démontée.

## Revendications

1. Injecteur d'implant (10) comportant:
- une cartouche de pliage (12) contenant un implant à injecter, la cartouche comprenant une canule (40) d'injection de l'implant et une excroissance radiale (50, 52) de liaison axiale ; et
- un mécanisme d'injection (14) comprenant un corps tubulaire (16) présentant à une extrémité des moyens d'accrochage de la cartouche (12), lesquels moyens d'accrochage de la cartouche (12) comportent une encoche (26) ménagée dans le corps tubulaire (16) et présentant à son extrémité débouchant à l'extrémité du corps (16) un passage étroit (26A) prolongé par une lumière évasée (26B) permettant une liaison par baïonnette de la cartouche (12) et du corps tubulaire (16),
**caractérisé en ce que** l'injecteur comporte un étui (32) de protection de la canule (40) dé la cartouche de pliage (12), lequel étui (32) comporte un boîtier (73) de réception de l'extrémité d'injection de la canule (40), et au moins une surface (78, 80) s'étendant radialement d'appui de l'excroissance radiale (50, 52) contre l'étui (32).

2. Injecteur selon la revendication 1, **caractérisé en ce que** la cartouche (12) de pliage comporte une pince formée de deux branches articulées (34, 36) présentant chacune une aile de manoeuvre (50, 52) propres à être accolées et **en ce que** ladite excroissance radiale est formée des deux ailes de manoeuvre accolées (50, 52).

3. Injecteur selon l'une-quelconque des revendications, **caractérisé en ce que** l'étui de protection (32) comporte deux saillies (72) et la canule (40) présente deux encoches (70) de positionnement propres à coopérer avec les saillies (72) pour le positionnement relatif de l'étui (34) et de la canule (40).

4. Injecteur selon l'une quelconque des revendications, **caractérisé en ce que** la longueur du boîtier (73) de l'étui (32) est supérieure à la longueur de la canule (40).

5. Injecteur selon l'une quelconque des revendications précédentes, **caractérisée en ce que** le boîtier (73) de réception de l'extrémité d'injection de la canule (40) comporte deux surfaces (78, 80) d'appui de l'excroissance radiale contre l'étui (32).

6. Injecteur selon les revendications 1 et 5, **caractérisé en ce que** les le boîtier (73) présente une ouverture (74) d'introduction de la canule (40) qui est bordée sur plus de la moitié de son pourtour par une lèvre (76) présentant à chaque extrémité une surface d'appui (78, 80).

7. Injecteur selon l'une quelconque des revendications précédentes, **caractérisé en ce que** la ou chaque surface d'appui (78, 80) s'étend autour du corps (16) lorsque la cartouche contenue dans l'étui (32) est engagée à l'avant du corps (16).

8. Injecteur selon l'une quelconque des revendications précédentes, **caractérisée en ce que** l'étui (32) est déplaçable angulairement par rapport au corps (12) lorsque la cartouche contenue dans l'étui (32) est engagée à l'avant du corps (16).

9. Injecteur selon l'une quelconque des revendications précédentes, **caractérisée en ce que** le corps (16) est dépourvu de bague rotative pour la mise en place de l'excroissance radiale (50, 52) dans l'encoche (26).

10. Injecteur selon l'une quelconque des revendications précédentes, **caractérisé en ce que** le corps comporte une languette élastique (26D) de sollicitation de l'excroissance radiale (50, 52) de la cartouche en regard d'un épaulement (26C) bordant la lumière évasée (26B) de l'encoche.

## Patentansprüche

1. Implantatinjektor (10), aufweisend:
- eine Faltkartusche (12), welche ein zu injizierendes Implantat enthält, wobei die Kartusche eine Kanüle (40) zum Injizieren des Implantats und einen Radialfortsatz (50, 52) zur axialen Verbindung aufweist, und
- einen Injektionsmechanismus (14), welcher einen rohrförmigen Körper (16) aufweist, der an einem Ende Mittel zur Kupplung der Kartusche (12) aufweist, welche Mittel zur Kupplung der Kartusche (12) einen Schlitz (26) aufweisen, der im rohrförmigen Körper (16) ausgebildet ist und der an seinem Ende, das am Ende des Körpers (16) ausmündet, eine schmale Passage (26A) hat, die von einem aufgeweiteten Kanal (26B) verlängert ist, der eine Bajonettverbindung der Kartusche (12) und des rohrförmigen Körpers (16) erlaubt,
**dadurch gekennzeichnet, dass** der Injektor ein Futteral (32) zum Schutz der Kanüle (40) der Faltkartusche (12) aufweist, welches Futteral (32) ein Gehäuse (73) zum Aufnehmen des Injektionsendes der Kanüle (40) und zumindest eine Fläche (78, 80), welche sich radial erstreckt zum Stützen des Radialfortsatzes (50, 52) gegen das Futteral (32), aufweist.

2. Injektor gemäß Anspruch 1, **dadurch gekennzeichnet, dass** die Faltkartusche (12) eine Zange aufweist, die von zwei gelenkverbundenen Armen (34, 36) gebildet ist, von welchen jeder einen Betätigungsflügel (50, 52) aufweist, die geeignet sind, um verbunden zu sein, und dadurch, dass der besagte Radialfortsatz von den zwei Betätigungsflügeln (50, 52), welche verbunden sind, gebildet ist.

3. Injektor gemäß irgendeinem der vorhergehenden Ansprüche, **dadurch gekennzeichnet, dass** das Futteral (32) zum Schutz zwei Vorsprünge (72) aufweist und die Kanüle (40) zwei Richtig-positionier-Schlitze (70) aufweist, um mit den Vorsprüngen (72) für die relative Positionierung des Futterals (34) und der Kanüle (40) zu kooperieren.

4. Injektor gemäß irgendeinem der vorhergehenden Ansprüche, **dadurch gekennzeichnet, dass** die Länge des Gehäuses (73) des Futterals (32) größer ist als die Länge der Kanüle (40).

5. Injektor gemäß irgendeinem der vorhergehenden Ansprüche, **dadurch gekennzeichnet, dass** das Gehäuse (73) zur Aufnahme des Injektionsendes der Kanüle (40) zwei Flächen (78, 80) zum Abstützen des Radialfortsatzes gegen das Futteral (32) aufweist.

6. Injektor gemäß den Ansprüchen 1 und 5, **dadurch gekennzeichnet, dass** das Gehäuse (73) eine Einführöffnung (74) für die Kanüle (40) aufweist, welche bis über die Hälfte ihres Umfangs von einer Lippe (76) begrenzt ist, die an jedem Ende eine Stützfläche (78, 80) aufweist.

7. Injektor gemäß irgendeinem der vorhergehenden Ansprüche, **dadurch gekennzeichnet, dass** sich die oder jede Stützfläche (78, 80) um den Körper (16) herum erstreckt, wenn die Kartusche, welche im Futteral (32) aufgenommen ist, mit dem Vorderteil des Körpers (16) im Eingriff ist.

8. Injektor gemäß irgendeinem der vorhergehenden Ansprüche, **dadurch gekennzeichnet, dass** das Futteral (32) mit Bezug auf den Körper (12) winkelmäßig verschiebbar ist, wenn die Kartusche, welche im Futteral (32) aufgenommen ist, mit dem Vorderteil des Körpers (16) im Eingriff ist.

9. Injektor gemäß irgendeinem der vorhergehenden Ansprüche, **dadurch gekennzeichnet, dass** der Körper (16) zur Positionierung des Radialfortsatzes (50, 52) in dem Schlitz (26) keinen drehbaren Ring hat.

10. Injektor gemäß irgendeinem der vorhergehenden Ansprüche, **dadurch gekennzeichnet, dass** der Körper eine elastische Raste (26D) zum Belasten des Radialfortsatzes (50, 52) der Kartusche mit Bezug auf einen Absatz (26C) aufweist, der an den aufgeweiteten Kanal (26B) des Schlitzes angrenzt.

## Claims

1. An implant injector comprising :
- a folding cartridge (12) containing an implant intended to be injected, the cartridge comprising an injection cannula (40) for the implant and a radial extension (50, 52) of axial connexion ; and
- an injection mechanism (14) comprising a tubular body (16) having, at one end, means for linking to the cartridge (12), said means for linking to the cartridge (12) including a notch (26) made in the tubular body (16) and having, at an end opening at the end of the body (16) a narrow gap (26A) extended by a flared opening (26B) allowing the cartridge (12) and the body (16) to be connected in a bayonet-type manner,
**characterized in that** the injector comprises a casing (32) for protecting the cannula (40) of the folding cartridge (12), said casing (32) including a housing (73) for the injection end of the cannula (40) and at least one bearing surface (78, 80) extending radially for the radial extension (50, 52) to rest against the casing (32).

2. An injector according to claim 1, **characterized in that** the folding cartridge (12) comprises a clamp formed by two articulated arms (34, 36), each of which having a maneuvering flange (50, 52) able to be applied one on the other, and **in that** said radial extension is formed by the two maneuvering flange (50, 52) applied one on the other.

3. An injector according to any one of the claims, **characterized in that** the casing (32) comprises two projections (72) and the cannula (40) has two positioning notches (70) able to cooperate with the projections (72) for relatively positioning the casing (34) and the cannula (40).

4. An injector according to any one of the claims, **characterized in that** the length of the housing (73) of the casing (32) is superior to the length of the cannula (40).

5. An injector according to any one of the preceding claims, **characterized in that** the housing (73) for the injection end of the cannula (40) comprises two bearing surfaces (78, 80) of the radial extension (50, 52) against the casing (32).

6. An injector according to claims 1 and 5, **characterized in that** the housing (73) has an opening (74) for introducing the cannula (40), which is bordered on more than half of its periphery by a lip (76) having, at each end, a bearing surface (78, 80).

7. An injector according to any one of the preceding claims, **characterized in that** the or each bearing surface (78, 80) extends around the body (16) when the cartridge contained in the casing (32) is engaged at the front of the body (16).

8. An injector according to any one of the preceding claims, **characterized in that** the casing (32) is angularly movable relative to the body (16) when the cartridge contained in the casing (32) is engaged at the front of the body (16).

9. An injector according to any one of the preceding claims, **characterized in that** the body (16) is devoid of a rotative ring for placing the radial extension (50, 52) into the notch (26).

10. An injector according to any one of the preceding claims, **characterized in that** the body (16) comprises an elastic tongue (26D) for soliciting the radial extension (50, 52) of the cartridge opposite a shoulder (26C) bordering the flared opening (26B) of the notch.
